# EUROPÄISCHE PATENTSCHRIFT

(11) **EP 2 461 785 B1**
(45) Veröffentlichungstag und Bekanntmachung des Hinweises auf die Patenterteilung: **13.11.2019**
(21) Anmeldenummer: 10736991.0
(22) Anmeldetag: 24.07.2010
(51) Int. Cl.: A61K 6/00, A61K 6/02, A61K 6/10, A61K 6/083

(54) **ANTIMIKROBIELL AUSGESTATTETE DENTALMATERIALIEN, INSBESONDERE ZUR VERHINDERUNG VON PLAQUEANLAGERUNGEN**
DENTAL MATERIALS CONTAINING ANTIMICROBIAL AGENTS, PARTICULARLY FOR THE PREVENTION OF PLAQUE DEPOSITS
MATÉRIAUX DENTAIRES DOTÉS DE PROPRIÉTÉS ANTIMICROBIENNENES, EN PARTICULIER POUR L'INHIBITION DE PLAQUE

(30) Priorität: 04.08.2009 DE 102009035970
(43) Veröffentlichungstag der Anmeldung: 13.06.2012
(73) Patentinhaber: Kulzer GmbH, 63450 Hanau (DE)
(72) Erfinder: RUPPERT, Klaus, 63477 Maintal (DE); BEYER, Mario, 61350 Bad Homburg (DE); VOGT, Sebastian, 99092 Erfurt (DE)
(74) Vertreter: Bendele, Tanja
(86) Internationale Anmeldenummer: PCT/EP2010/004551
(87) Internationale Veröffentlichungsnummer: WO 2011/015293

(56) Entgegenhaltungen:
- EP-A1- 2 113 237
- WO-A2-99/07326
- DE-A1- 10 332 680
- DE-A1-102007 040 569
- US-A- 5 300 290
- US-A1- 2006 205 838

## Beschreibung

Die Erfindung betrifft antimikrobiell ausgestattete Dentalmaterialien, insbesondere zur Verhinderung von Plaqueanlagerungen.

Polymere Dentalmaterialien, insbesondere auf Acrylat-/Methacrylat-Basis, welche zum dauerhaften Verbleib in die Mundhöhle eingebracht werden, neigen bei mangelnder Oralhygiene zur Plaquebesiedelung an der Werkstoffoberfläche.

Plaque setzt sich aus verschiedenen Bakterien und Hefen zusammen, welche sich durch Proteine und Kohlenhydrate fest auf Oberflächen wie z.B. Zähnen oder dentalen Werkstoffen verankern. Auf dieser ersten Bakterienschicht können sich dann weitere Bakterien festsetzen und damit eine dreidimensionale Kolonie bilden. Durch bestimmte Substanzen, welche von den Bakterien abgegeben werden, ist dieser "Biofilm" nahezu unangreifbar für Antibiotika.
Neben dem hygienischen Aspekt führt Plaque im fortgeschrittenen Stadium auch zu einer starken Verfärbung, so dass es zu ästhetischen Beeinträchtigungen kommt.

### Stand der Technik

Zur Verminderung der Plaque-Besiedlung bei Dentalwerkstoffen kommen verschiedene Möglichkeiten in Frage: Einsatz von Mikrobiziden, eine z. B. auf Poly(ethylenglykogen) basierende, proteinabweisende Oberfläche oder eine hydrophobe Beschichtung der Dentalwerkstoffe, welche die Anhaftung der Bakterien auf dem Werkstoff erschwert.

Die Verwendung von quaternären Ammoniumsalzen als antimikrobielle Additive ist seit langem bekannt. So wird z.B. ein Silan mit quaternären Ammoniumgruppen als funktionelle Gruppe von der Fa. Microbeshield hergestellt und zur antibakteriellen Ausrüstung von Filtern, Textilien und Wundauflagen vermarktet. In GB 1433303 A werden Füllerpartikel für Kunststoffe beschrieben, die silanisiert und mit quaternären Ammoniumsalzen beschichtet werden. So behandelte Diatomeenerde oder pyrogene Kieselsäuren werden beispielsweise zur Verwendung in Holzbeschichtungen, Dichtungsmassen, Kathetern oder Textilfasern vorgeschlagen.

Ferner sind Additive auf der Basis von kationischen Oligomeren bekannt (Akacid®, Fa. PoC) sowie silberhaltige Additive [silberhaltige Gläser, Salze, Zeolithe (US 6,436,422 B1)]. Wenn man derartige Beschichtungen auf Füllerpartikeln im Nanometerbereich, wie sie für Dentalwerkstoffe gängig sind, aufbringt, ergibt dies ein Material mit geringer Abrasionsstabilität gegenüber den natürlichen Vorgängen bei der Nahrungsaufnahme durch das Kauen. So ausgestattete Dentallacke oder Fissurenversiegler müssen daher wiederholt aufgetragen werden oder scheiden generell als proteinabweisende Schichten aus. Bei der Verwendung von mikro- oder nanoskaligem, metallischem Silber ist die Gewährleistung einer natürlichen Farbgebung von Zahnfleischimitat, Füllungen, Verblendungen bzw. künstlichem Zahnmaterial selbst nicht gegeben. Die silberhaltigen Materialen erfahren eine gelb bis gräuliche Farbgebung, abhängig von der Korngröße des verwendeten Edelmetalls.

JP 10025218 A beschreibt anorganische Füllstoffe, die mit einer antimikrobielle Gruppen enthaltenden Polymerschicht überzogen sind. Die Schicht entsteht durch Polymerisation entsprechender (Meth)acrylat-Monomere, die Phosphonium- oder quaternäre Ammoniumgruppen tragen.

Gemäß DE 10 2005 042 078 A1 werden dentale Füllkörper mit antimikrobiell wirksamen Polysacchariden überzogen. Derartige mit einem Polysaccharid beschichtete Füllkörper werden mit einem weiteren Polymer umhüllt (Seite 4, Absatz 0034). In das Polysaccharid Chitosan wird noch eine CC-Doppelbindung eingeführt, um eine Einpolymerisation in das Polymer zu ermöglichen (Seite 4, Absatz 0037). Das Chitosan wird entsprechend auf der Füllkörperoberfläche kovalent gebunden (Seite 5, Absatz 0047).

### Aufgabenstellung

Aufgabe der vorliegenden Erfindung ist es, ein Verfahren bereitzustellen, mit dem sich die Besiedelung von Plaque auf dentalen Werkstoffen dauerhaft verhindern bzw. verzögern lässt, ohne die Produkteigenschaften des Dentalmaterials negativ zu beeinflussen.

Hierbei sind folgende Kernanforderungen wichtig, von denen mehrere erfüllt sein sollten:
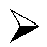 Eine homogene Verteilung des Wirkstoffs im Bulk-Material und auf der Materialoberfläche sollte gewährleistet sein, also keine punktuelle Verteilung.
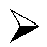 Der Werkstoff darf nach der Wirkstoff-Freisetzung keine Mikro- und Makroporen aufweisen. Das ist sowohl aus Gründen der Ästhetik wichtig als auch deshalb, weil jede Rissbildung ein Ausgangspunkt von Neubesiedlung mit Plaque ist.
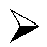 Eine Inaktivierung des Wirkstoffs auf der Oberfläche soll erschwert sein. Dies ist zweckmäßig durch Nachdiffusion des Wirkstoffs aus dem Bulk-Material zu erreichen.
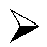 Ein breites Wirkungsspektrum gegen typische Oralkeime (bakterizid sowohl gegen gram-positive wie gram-negative Keime);
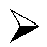 gegebenenfalls zusätzlich fungizide Eigenschaften.
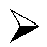 Der Wirkstoff soll retardierend freigesetzt werden.
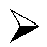 Der Wirkstoff soll farb- und geruchlos sein.
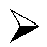 Der Wirkstoff soll eine so hohe Freisetzungsrate haben, dass eine antimikrobielle Wirksamkeit gegeben ist, jedoch keine toxischen oder irritierenden/sensibilisierenden Effekte auftreten.
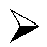 Der Wirkstoff darf die Polymerisation des Produktes nicht stören und die Werkstoffeigenschaften negativ beeinflussen, es darf keine Phasenseparation (Weichmachereffekt) auftreten.
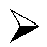 Der Wirkstoff darf nur eine geringwahrscheinliche Resistenzbildung gegenüber den typischen Oralkeimen aufweisen.
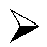 Keine chemischen Reaktionen mit den Monomeren, Füllstoffen, Initiatoren, Stabilisatoren und Farbstoffen während der Lagerung und Härtungsreaktion.

Die Aufgabe wird durch Zusammensetzungen nach Anspruch 1 gelöst. Weitere Ausführungsformensind den abhängigen Ansprüchen 2 und 3 zu entnehmen.

Bevorzugte Wirkstoffe sind folgende:
aus: Majic-Todt, Ante, Dissertation 2003, "Prüfung von Lavasept® auf antiseptische Aktivität und Plaquehemmung"

| **Wirkstoffgruppe** | **Antiseptikum** |
|---|---|
| Halogene | PVP-Iod, Natriumhypochlorit, Tosylchloramidnatrium (Chloramin T) |
| Guanidine | Chlorhexidin, Alexidin |
| Kationenaktive Verbindungen | Cetylpyridiniumchlorid |
| Quarternäre Ammoniumverbindungen | Benzalkoniumchlorid |
| Pyrimidine | Hexetidin |
| Bispyridine | Octenidindihydrochlorid |
| Diphenylether | Triclosan |

Die Zusammensetzungen der Erfindung eignen sich zur Verwendung in oder als: Füllungskomposite, Verblendkomposite, Prothesenwerkstoffe, künstliche Zähne, Abformmassen, Schutzlacke, Fissurenversiegler, Dentinbondings sowie für Hufmaterial in der Veterinärmedizin.
Zur Herstellung der erfindungsgemäßen antimikrobiell ausgestatteten Materialien wird der Wirkstoff in einem geeigneten Lösungsmittel gelöst, die Lösung mit dem anorganischen Füllstoff bzw. organischem Polymer vermischt und das Lösungsmittel anschließend restlos entfernt.
In erfindungsgemäßen Dentalkompositen werden vornehmlich radikalisch polymerisierbare Monomere aus der Gruppe der Methacrylate und Acrylate eingesetzt:
Bekannte für Dentalmaterial geeignete viskose Harze/Monomere sind Polyurethandimethacrylat (PUDMA), Diurethandimethacrylate (DUDMA), und/oder Polycarbonatdimethacrylat (PCDMA, Kondensationsprodukt aus 2 Teilen Hydroxyalkylmethacrylat und 1 Teil Bis(chloroformat), wie in US 5,276,068 und 5,444,104 beschrieben, ferner ethoxyliertes Bisphenol A dimethacrylate (EBPDMA), wie in US 6,013,694 beschrieben; und besonders Bis-GMA (Bowen Monomer). Verdünnermonomere werden zur Viskositätssenkung aber auch zur Beeinflussung der Benetzbarkeit eingesetzt. Beispiele für geeignete Verdünnermonomere sind Hydroxyalkyl(meth)acrylate wie 2-Hydroxy-ethyl(meth)acrylat, 2-Hydroxypropyl (meth)acrylat und 4-Hydroxybutyl(meth)acrylat; Ethylen-glycol Einheiten enthaltende (Meth)acrylate wie Ethylenglycolmethacrylat, Diethyleneglycol-methacrylat, Triethylenglycoldimethacrylat und Tetraethylenglycoldimethacrylat; Diol-Dimeth-acrylate wie 1,4-Butanedioldi(meth)acrylat, Dodecandioldi(meth)acrylat und 1,6-Hexan-dioldi(meth)acrylat, davon besonders 1,6-Hexandioldimethacrylat (HDDMA). Weitere geeignete Monomere sind z. B. Polyethyleneglycolmono(meth)acrylat; Glyceroldi(meth)acrylat; Trimethylolpropandi(meth)acrylat; Pentaerythritoltri(meth)acrylat; das (Meth)acrylat des Phenylglycidylethers. Tri(ethylene glycol)dimethacrylat (TEGDMA) ist besonders bevorzugt.

Die Einsatzmengen der Verdünnermonomere und der viskosen Harze können in weiten Grenzen schwanken und bewegen sich um 1 bis 70 Gewichtsprozent bezogen auf das Dentalmaterial.

Vernetzer werden beispielsweise zur Erhöhung der Endfestigkeit zugesetzt:
Als difunktionelle (Meth)acrylat-Vernetzer eignen sich vor allem vernetzende bi- oder mehrfunktionelle Acrylate bzw. Methacrylate wie z.B. Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)-acrylat, sowie 1,4-Butandioldi(meth)acrylat, 1,10-Decandioldi(meth)acrylat oder 1,12-Dodecan-dioldi(meth)acrylat.

Vorteilhaft kann auch die Beimischung bekannter schrumpfungsarmen radikalisch ringöffnend polymerisierbarer Monomere wie z.B. mono- oder multifunktioneller Vinylcyclopropane bzw. bicyclischer Cyclopropanacrylatderivate (vgl. DE 196 16 183 C2 bzw. EP 03 022 855) oder cyclischer Allylsulfide (vgl. US 6,043,361 oder US 6,344,556) sein, die darüber hinaus auch in Kombination mit den voranstehend aufgeführten Di(meth)acrylat-Vernetzern verwendet werden können. Bevorzugte ringöffnend polymerisierbare Monomere sind solche Vinylcyclopropane, wie 1,1-Di(ethoxycarbonyl)- oder 1,1-Di(methoxycarbonyl)-2-vinylcyclopropan oder die Ester der 1-Ethoxycarbonyl- oder 1-Methoxycarbonyl-2-vinylcyclopropancarbonsäure mit Ethylenglycol, 1,1,1-Trimethylolpropan, 1,4-Cyclohexandiol oder Resorcin. Bevorzugte bicyclische Cyclopropanderivate sind 2-(Bicyclo[3.1.0]hex-1-yl)acrylsäuremethyl- oder -ethylester oder deren Disubstitutionsprodukte in 3-Stellung wie (3,3-Bis(ethoxycarbonyl)bicyclo[3.1,0]hex-1-yl)acryl-säuremethyl- oder -ethylester. Bevorzugte cyclische Allylsulfide sind vor allem die Additionsprodukte von 2-(Hydroxymethyl)-6-methylen-1,4-dithiepan oder 7-Hydroxy-3-methylen-1,5-dithiacylooctan mit 2,2,4-Trimethylhexamethylen-1,6-diisocyanat oder dem asymmetrischen Hexamethylendiisocyanat-Trimeren Desmodur ® VP IS 2294 der Bayer AG.

Geeignet sind auch kationisch polymerisierbare Calix[n]arene entsprechend der allgemeinen Formel (I) wie in DE102007035734A1 beschrieben.

Bekannte schrumpfungsarme kationisch ringöffnend polymerisierbare Monomere sind z.B. Glycidylether oder cycloaliphatische Epoxide, cyclische Ketenacetale, Spiroorthocarbonate, Oxetane oder bicyclische Orthoester.

Beispiele sind: 2-Methylen-1,4,6-trioxaspiro[2.2]nonan, 3,9-Dimethylen-1,5,7,11-tetraoxaspiro[5.5]undecan, 2-Methylen-1,3-dioxepan, 2-Phenyl-4-methylen-1,3-dioxolan, Bisphenol-A-diglycidylether, 3,4-Epoxy-cyclohexylmethyl-3,4-epoxycyclohexancarboxylat, Bis(3,4-epoxycyclohexylmethyl)adipat, Vinylcyclohexendioxid, 3-Ethyl-3-hydroxymethyloxetan, 1,10-Decandiyl-bis-(oxymethylen)-bis-(3-ethyloxetan) oder 3,3-(4-Xylylendioxy)-bis-(methyl-3-ethyloxetan) bzw. weitere in der EP 0 879 257 B1 genannte Epoxide. Als kationisch polymerisierbare Matrixsysteme eignen sich auch Kieselsäurepolykondensate, die beispielsweise durch hydrolytische Kondensation von Silanen, die kationisch polymerisierbare Gruppen, bevorzugt z.B. Epoxid-, Oxetan-, oder Spiroorthoestergruppen tragen. Solche Kieselsäurepolykondensate sind beispielsweise in der DE 41 33 494 C2 oder US 6,096,903 beschrieben.

Die erfindungsgemäßen Dentalmaterialien auf der Basis radikalisch polymerisierbarer Monomere lassen sich mit den bekannten radikalischen Initiatoren (vgl. Encyclopedia of Polymer Science and Engineering, Vol. 13, Wiley-Intersci. Pub., New York etc. 1988, 754ff.) polymerisieren.

Es eignen sich besonders Photoinitiatoren (vgl. J.P. Fouassier, J.F. Rabek (Hrsg.), Radiation Curing in Polymer Science and Technology, Vol. II, Elsevier Applied Science, London und New York 1993) für den UV- oder sichtbaren Bereich, wie z. B. Benzoinether, Dialkylbenzilketale, Dialkoxyacetophenone, Acyl- der Bisacylphosphinoxide, [alpha]-Diketone wie 9,10-Phenanthrenchinon, Diacetyl, Furil, Anisil, 4,4'-Dichlorbenzil und 4,4'-Dialkoxybenzil und Campherchinon.

Weiterhin können auch Azoverbindungen, wie 2,2'-Azobis(isobutyronitril) (AIBN) oder Azobis-(4-cyanovaleriansäure), oder Peroxide, wie Dibenzoylperoxid, Dilauroylperoxid, tert-Butylperoctoat, tert-Butylperbenzoat oder Di-(tert-butyl)-peroxid verwendet werden. Als Initiatoren für die Heißhärtung eignen sich Benzpinakol und 2,2'-Dialkylbenzpinakole.

Zur Beschleunigung der Initiierung mittels Peroxiden oder [alpha]-Diketonen werden häufig deren Kombinationen mit aromatischen Aminen eingesetzt. Redoxsysteme, die sich bereits bewährt haben, sind: Kombinationen aus Benzoylperoxid oder Campherchinon mit Aminen, wie N,N-Dimethyl-p-toluidin, N,N-Dihydroxyethyl-p-toluidin, p-Dimethylaminobenzoesäureethylester oder strukturverwandte Systeme. Darüber hinaus sind auch Redoxsysteme bestehend aus
Peroxiden und solchen Reduktionsmitteln, wie z. B. Ascorbinsäure, Barbiturate oder Sulfinsäuren, geeignet.

Erfindungsgemäße Dentalmaterialien auf der Basis kationisch polymerisierbarer Monomere lassen sich mit den bekannten kationischen Photoinitiatoren, besonders mit Diaryliodonium- oder Triarylsulfoniumsalzen, ggf. in Gegenwart geeigneter Sensibilisatoren, wie z.B. Campherchinon, aushärten. Beispiele für geeignete Diaryliodoniumsalze, die mit Campherchinon oder Thioxanthonen als Sensibilisator im sichtbaren Bereich eingesetzt werden können, sind die kommerziell zugänglichen 4-Octyloxy-phenyl-phenyl-iodoniumhexafluoroantimonat oder Isopropylphenyl-methylphenyl-iodoniumtetrakis (pentafluorophenyl)borat.

Weiterhin können erfindungsgemäße Dentalmaterialien einen oder mehrere Füllstoffe enthalten, vorzugsweise organische oder anorganische partikuläre Füllstoffe. Bevorzugte anorganische partikulären Füllstoffe sind amorphe kugelförmige nanopartikuläre Füllstoffe auf der Basis von Oxiden, wie pyrogene Kieselsäure oder Fällungskieselsäure, ZrO₂ und TiO₂ oder Mischoxide aus SiO₂, ZrO₂ und/oder TiO₂ mit einem mittleren Partikeldurchmesser von 10 bis 200 nm, Minifüllstoffe, wie Quarz-, Glaskeramik- oder Glaspulver mit einer durchschnittlichen Teilchengrösse von 0,2 bis 5 [mu]m sowie röntgenopake Füllstoffe, wie Ytterbiumtrifluorid oder nanopartikuläres Tantal(V)-oxid bzw. Bariumsulfat. Darüber hinaus können auch faserförmige Füllstoffe wie Glasfasern, Polyamid- oder Kohlenstoff-Fasern eingesetzt werden.

Gegenstand der Erfindung ist auch ein Dentalmaterial aus der Gruppe der Komposite, Prothesenbasismaterialien, Adhäsive, Lacke und Fissurenversiegler mit antimikrobiellen Eigenschaften ausgestatteten anorganischen Partikeln als Dentalfüllstoff, wobei der Dentalfüllstoff der Gruppe bestehend aus einem Dentalglas, Barium-Aluminium-Silikat-Glas, SiO₂, ZrO₂ und YbF₃ angehört, in dem der Dentalfüllstoff als Trägerstoff dient. Erfindungsgemäßes Dentalmaterial kann anorganische Trägerstoffe mit einem Durchmesser kleiner 50 µm, insbesondere kleiner 10 µm, bevorzugt kleiner 2 µm aufweisen.

Der erfindungsgemäße anorganische Trägerstoff kann aus SiO₂ bestehen. Ferner kann der erfindungsgemäße anorganische Trägerstoff aus SiO₂ und einem weiteren Schwermetalloxid wie z.B. ZrO₂ bestehen. Der erfindungsgemäße anorganische Trägerstoff kann zusätzlich noch eine funktionalisierte Oberfläche zur Löslichkeitsvermittlung aufweisen.

Offenbart werden organische Trägerstoffe, die aus PMMA oder einem Methacrylatcopolymer bestehen.

Ein erfindungsgemäßes Dentalmaterial kann Additive zu bis zu 6 Gew.-% oder zu bis zu 3 Gew.-% aufweisen.

Im Dentalmaterial kann noch eine weitere antimikrobielle Komponente homogen verteilt sein. Ferner kann die weitere antimikrobielle Komponente der Gruppe bestehend aus monokationischen Antiseptika, dikationischen Antiseptika, oligo- oder polymere kationischen Antiseptika und antiseptischen Schwermetall-Verbindungen angehören.

Schließlich lassen sich den erfindungsgemäßen Dentalmaterialien im Bedarfsfalle weitere Additive zusetzen, wie z.B. Stabilisatoren, UV-Absorber, Farbstoffe oder Pigmente sowie Lösungsmittel, wie z.B. Wasser, Ethanol, Aceton oder Ethylacetat oder Gleitmittel.

Dabei sind die erfindungsgemäßen Dentalmaterialien je nach Verwendungszweck vorzugsweise aus folgenden Komponenten zusammengesetzt:
Erfindungsgemäße Zemente enthalten bevorzugt: (a) 0,5 bis 30 Gew.-%, besonders bevorzugt 0,5 bis 20 Gew.-% mindestens eines polymerisierbaren Calix[n]arens gemäß Formel (I), (b) 0,01 bis 2 Gew.-%, besonders bevorzugt 0,01 bis 1,5 Gew.-% Initiator, (c) 1 bis 30 Gew.-%, besonders bevorzugt 5 bis 20 Gew.-% mindestens eines weiteren kationisch und/oder
   radikalisch polymerisierbaren Monomers und/oder eines weiteren ringöffnend polymerisierbaren
Monomers, bevorzugt eines mehrfach funktionellen (Meth)acrylats, (d) 5 bis 70 Gew.-%, besonders bevorzugt 10 bis 60 Gew.-% Füllstoff und (e) 0,01 bis 5 Gew.-%, bevorzugt 0,01 bis 2 Gew.-%, besonders bevorzugt 0,01 bis 1 Gew.-% Additiv, wobei sich die Prozentangaben jeweils auf 100 % addieren.

Erfindungsgemäße Füllungskomposite enthalten bevorzugt: (a) 0,5 bis 30 Gew.-%, besonders bevorzugt 0,5 bis 20 Gew.-% mindestens eines polymerisierbaren Calix[n]arens gemäß Formel (I), (b) 0,01 bis 5 Gew.-%, bevorzugt 0,01 bis 2 Gew.-%, besonders bevorzugt 0,01 bis 1,5 Gew.-% Initiator, (c) 1 bis 30 Gew.-%, bevorzugt 5 bis 20 Gew.-% besonders bevorzugt 5 bis 15 Gew.-% mindestens eines weiteren kationisch und/oder radikalisch polymerisierbaren Monomers und/oder mindestens eines weiteren ringöffnend polymerisierbaren Monomers, besonders bevorzugt ins mehrfach funktionellen (Meth)acrylats (d) 5 bis 85 Gew.-%, besonders bevorzugt 10 bis 80 Gew.-% Füllstoff und (e) 0,01 bis 5 Gew.-%, bevorzugt 0,01 bis 3 Gew.-%, besonders bevorzugt 0,01 bis 2 Gew.-% Additiv, wobei sich die Prozentangaben jeweils auf 100 Gew.-% addieren.

Erfindungsgemäße Beschichtungsmaterialien enthalten bevorzugt: (a) 1 bis 70 Gew.-%, besonders bevorzugt 1 bis 50 Gew.-% mindestens eines polymerisierbaren Calix[n]arens gemäß Formel (I), (b) 0,01 bis 5 Gew.-%, bevorzugt 0,01 bis 2 Gew.-%, besonders bevorzugt 0,1 bis 1,5 Gew.-% Initiator, (c) 5 bis 70 Gew.-%, bevorzugt 5 bis 60 Gew.-%, besonders bevorzugt
5 bis 50 Gew.-% mindestens eines weiteren kationisch und/oder radikalisch polymerisierbaren Monomers, und/oder mindestens eines weiteren ringöffnend polymerisierbaren Monomers, besonders bevorzugt mindestens eines mehrfach funktionellen (Meth)acrylats, (d) 1 bis 30 Gew.-%, bevorzugt 3 bis 20 Gew.-%, besonders bevorzugt 3 bis 15 Gew.-% eines Füllstoffs, bevorzugt eines nanopartikulären Füllstoffs und (e) 0,01 bis 5 Gew.-%, bevorzugt 0,01 bis 3 Gew.-%, besonders bevorzugt 0,01 bis 2 Gew.-%, ganz besonders bevorzugt 0,01 bis 1 Gew.-% Additiv (f) 0 bis 70 Gew.-%, besonders bevorzugt 0 bis 30 Gew.-% Lösungsmittel, wobei sich die Prozentangaben jeweils auf 100 % addieren.

Erfindungsgemäße Dentaladhäsive enthalten bevorzugt: (a) 0,5 bis 50 Gew.-%, besonders bevorzugt 1,0 bis 30 Gew.-% mindestens eines polymerisierbaren Calix[n]arens gemäß Formel (I), (b) 0,01 bis 5 Gew.-%, besonders bevorzugt 0,01 bis 2 Gew.-% mindestens eines Initiators (c) 5 bis 70 Gew.-%, besonders bevorzugt 5 bis 60 Gew.-% mindestens eines weiteren kationisch und/oder radikalisch polymerisierbaren Monomers und/oder mindestens ein ringöffnend polymersierbaren Monomers, besonders bevorzugt mindestens eines mehrfach funktionellen (Meth)acrylats, (d) 0 bis 30 Gew.-%, besonders bevorzugt 3 bis 20 Gew.-% Füllstoff und (e) 0,01 bis 5 Gew.-%, besonders bevorzugt 0,01 bis 3 Gew.-% Additive, (f) 0 bis 50 Gew.-%, besonders bevorzugt 0 bis 20 Gew.-% Lösungsmittel, wobei sich die Prozentangaben auf 100 % addieren.

Erfindungsgemäße Prothesenbasismaterialien sind bevorzugt solche auf der Basis von Methacrylaten. Für ein Prothesenbasismaterial erwünschte Eigenschaften werden z.B. durch einen hohen Vernetzungsgrad oder durch den Zusatz von Schlagzähigkeitsverbesserern wie Polybutadien-Polymeren angestrebt, wie etwa in EP 1 702 633 A2 beschrieben. Prothesenbasismaterialien enthalten für gewöhnlich Pigmente zur Erzeugung eines zahnfleischähnlichen Eindrucks.

Das Material für künstliche Zähne unterliegt sehr ähnlichen Anforderungen wie die oben beschriebenen Füllungsmaterialien und ist daher aus den gleichen Komponenten aufgebaut.

Hufmaterialien sind ähnlich wie Prothesenbasismaterial zweikomponentige, selbsthärtende Kunststoffe, die zur Reparatur von Hufschäden bei Huftieren eingesetzt werden.

Für die Otoplastik geeignetes Material ist solches, das auch für Dentalabformmassen verwendet wird. Es handelt sich zumeist um additions- oder kondensationsvernetzende Silikonmaterialien, wie z.B. in EP 1 374 915 A2 beschrieben.

Die folgenden Beispiele erläutern die Erfindung näher. Teile- und Prozentangaben beziehen sich wie in der übrigen Beschreibung auf das Gewicht, sofern nicht anders angegeben.

### Beispiel 1: Antimikrobielle Ausstattung von Partikeln

3 g Octenidin-Dihydrochlorid werden in 97 g Ethanol gelöst. Die Lösung wird mit 8 g Aerosil OX 50 versetzt und bei Raumtemperatur intensiv gerührt. Durch leichtes Erwärmen auf ca. 40 - 45 °C wird unter weiterem Rühren der Hauptteil des Lösungsmittels entfernt. Die vollständige Entfernung geschieht durch leichtes Erwärmen bei Unterdruck (ca. 1 - 10 mbar).

### Beispiel 2: Dentalmaterialien mit Partikeln nach Beispiel 1

Aus Bis-GMA und TEDMA wird unter leichter Erwärmung und unter leichtem Rühren eine 70/30 - Mischung hergestellt. Es erfolgt die Zugabe von üblichen Photoinitiatoren und Stabilisatoren sowie 65 Gew.-% Dentalglas in einer Korngröße von ∼ 1 µm. Zur Einstellung der Rheologie werden 8 Gew.-% pyrogene Kieselsäure Aerosil OX50, behandelt mit Octenidin-Dihydrochlorid, zugegeben. Ferner werden nach Bedarf Farbpigmente zur Farbeinstellung zudosiert.

Es ergibt sich ein antimikrobiell ausgestattetes Dentalmaterial.

## Patentansprüche

1. Dentalmaterial aus der Gruppe der Komposite, Prothesenbasismaterialien, Adhäsive, Lacke und Fissurenversiegler mit mit antimikrobiellen Eigenschaften ausgestatteten anorganischen Partikeln als Dentalfüllstoff, wobei auf den anorganischen Partikeln mindestens ein antimikrobieller Wirkstoff aufgebracht ist, der an die Partikel über ionische Wechselwirkung oder Van-der-Waals-Wechselwirkung - aus der Gruppe der Dipol-Dipol-, Dipol-induzierter Dipol-, induzierter Dipol-induzierter Dipol-Wechselwirkungen - adsorbiert oder gebunden ist, wobei der Wirkstoff aus der Gruppe bestehend aus Iminopyridinium-Derivaten, Octenidin-Salzen, Dequalinium-Salzen, Sanguinarin und Poly-[2-(2-ethoxy)-ethoxyethyl-guanidiniumchlorid] ausgewählt ist, und wobei der Dentalfüllstoff der Gruppe bestehend aus einem Dentalglas, Barium-Aluminium-Silikat-Glas, SiO₂, ZrO₂ und YbF₃ angehört.

2. Dentalmaterial nach Anspruch 1, **dadurch gekennzeichnet, dass** Additive zu bis zu 6 Gew.-% oder zu bis zu 3 Gew.-% darin vorliegen.

3. Dentalmaterial nach Anspruch 1 oder 2, **dadurch gekennzeichnet, dass** im Dentalmaterial noch eine weitere antimikrobielle Komponente homogen verteilt ist.

4. Dentalmaterial nach Anspruch 3, wobei die weitere antimikrobielle Komponente der Gruppe bestehend aus monokationischen Antiseptika, dikationischen Antiseptika, oligo- oder polymeren kationischen Antiseptika und antiseptischen Schwermetall-Verbindungen angehört.

## Claims

1. Dental material from the group of composites, prosthetic base materials, adhesives, varnishes and fissure sealants comprising inorganic particles as dental filling materials having antimicrobial properties, at least one antimicrobial agent being applied onto the inorganic particles which is adsorbed or bound to the particles via ionic interaction or van der Waals interaction - from the group of dipole-dipole, dipole-induced dipole, induced dipole-induced dipole interactions, the agent being selected from the group consisting of iminopyridinium derivatives, octenidine salts, dequalinium salts, sanguinarine and poly-[2-(2-ethoxy)-ethoxyethyl)guanidinium chloride], and the dental filling material belonging to the group consisting of a dental glass, barium-aluminum-silicate glass, SiO₂, ZrO₂ and YbF₃.

2. Dental material according to claim 1, **characterised in that** additives are present therein at up to 6 % by weight or up to 3 % by weight.

3. Dental material according to claim 1 or 2, **characterised in that** one further antimicrobial component is homogeneously distributed in the dental material.

4. Dental material according to claim 3, wherein the further antimicrobial component belongs to the group consisting of monocationic antiseptic agents, dicationic antiseptic agents, oligo- or polymeric cationic antiseptic agents, and antiseptic heavy metal compounds.

## Revendications

1. Matériau dentaire du groupe des composites, des matériaux de base prothétique, des adhésifs, des vernis et des scellants de fissures comprenant des particules inorganiques comme matériaux de remplissage dentaire ayant des propriétés antimicrobiennes, au moins un agent antimicrobien étant appliqué sur les particules inorganiques qui est adsorbé ou lié à les particules par l'interaction ionique ou l'interaction de van der Waals - du groupe des interactions dipôle-dipôle, dipôle-dipôle induit, dipôle induit-dipôle induit, l'agent étant sélectionné parmi le groupe consistant en les dérivés iminopyridinium, les sels octénidine, les sels déqualinium, le sanguinarine, et le poly-[chlorure de 2-(2-éthoxy)-éthoxyéthyle)guanidinium], et le matériau de remplissage dentaire appartient au groupe consistant en un verre dentaire, un verre de silicate de baryum d'aluminium, SiO₂, ZrO₂ et YbF₃.

2. Matériau dentaire selon la revendication 1, **caractérisé en ce que** des additifs y sont présents jusqu'à 6 % en poids ou jusqu'à 3 % en poids.

3. Matériau dentaire selon la revendication 1 ou 2, **caractérisé en ce que** un autre composant antimicrobien est homogènement distribué dans le matériau dentaire.

4. Matériau dentaire selon la revendication 3, selon lequel l'autre composant antimicrobien appartient au groupe consistant en des agents antiseptiques monocationiques, des agents antiseptiques dicationiques, des agents antiseptiques cationiques oligo ou polymère, et des composés de métal lourd antiseptiques.
